# EUROPEAN PATENT SPECIFICATION

(11) **EP 1 443 317 B1**
(45) Date of publication and mention of the grant of the patent: **17.09.2008**
(21) Application number: 04250523.0
(22) Date of filing: 30.01.2004
(51) Int. Cl.: G01N 3/10, G01N 33/10

(54) **Apparatus and method for the rheological and mechanical analysis of materials**
Verfahren und Vorrichtung zur rheologische und mechanische Materialanalyse
Procédé et dispositif pour l'analyse rhéologique et mécanique de matériaux

(30) Priority: 31.01.2003 GB 0302297
(43) Date of publication of application: 04.08.2004
(73) Proprietor: UNITED BISCUITS (UK) LIMITED, Hayes, Middlesex UB4 8EE (GB)
(72) Inventor: Cicerelli, Lucio, High Wycombe HBuckinghamshire HP13 6AJ (GB); Celot, Frederico, High Wycombe HBuckinghamshire HP12 4JR (GB); De Cindio, Bruno, P Bucci I-87030 Arcevacota di Rende (IT); Migliori, Massimo, I-87100 Cosenza (IT); Gabriele, Domenico, I-87100 Cosenza (IT)
(74) Representative: Humphreys, Ceris Anne

(56) References cited:
- US-A- 1 468 900
- US-A- 3 332 281
- US-A- 3 580 050
- US-A- 4 838 081
- PATENT ABSTRACTS OF JAPAN vol. 0135, no. 66 (P-976), 15 December 1989 (1989-12-15) & JP 1 237430 A (TOYOTA CENTRAL RES & DEV LAB INC), 21 September 1989 (1989-09-21)

## Description

The invention relates to the rheological and mechanical analysis of materials, for example, flours and doughs. The invention comprises new apparatus for the analysis of materials having viscoelastic properties, especially doughs, a method of carrying out such analyses, and use of data obtained from such analyses, for example, in the control of baking processes or the characterisation of flour.

Manufacturers of dough products, especially baked products such as biscuits have traditionally assessed dough quality by hand, for example, by kneading and stretching the dough and by consciously or unconsciously applying empirical rules that relate the feel of a dough to the qualities of the final baked product. More recently there has been the development of the science of dough rheology and the invention of various tests and apparatus in an attempt to define and predict dough quality in a more scientific fashion.

Raw dough behaves as a viscoelastic material. That is, its rheological behaviour exhibits both elastic and viscous components. The balance between the viscous and elastic properties of a dough has an important influence on the properties of the final baked product. If a dough flows too much after mixing it may not maintain its desired final shape. If the elastic component of a dough is too dominant (often termed a "bucky" dough), it may be difficult to shape in the first place. Viscoelastic properties of a dough are also important when the product incorporates carbon dioxide bubbles produced by a raising agent. If the dough is insufficiently elastic, fewer bubbles will be trapped. This may result in a denser and less palatable bakery product. A denser product may also be more expensive to produce per unit volume.

Dough behaviour during sheeting, moulding, cutting and baking are also influenced by the viscoelastic properties of dough.

The unique viscoelastic properties of dough are largely due to gluten. Gluten is formed from protein found in grain endosperm and water. Grain endosperm contains proteins, largely for storage, that may be placed into 4 classes. The first two classes are albumins and globulins. Proteins in those classes are water-soluble and do not contribute significantly to the formation of gluten. Gliadin and glutenin are two water insoluble protein classes, and do contribute to gluten. The changes in the viscoelastic properties of dough that occur during dough development may be explained by the properties of the long gluten molecules, especially those derived from glutenin. Kneading, sheeting or mixing may cause neighbouring gluten molecules to align and thereby make the dough more elastic. If a dough is "over-developed" its elasticity may be diminished. The loss of elasticity in over-developed dough is thought to occur due to a net loss of disulphide bonds between and within parallel chains of gluten.

Strong flours may be used to make doughs that resist over-development more successfully than do doughs made from weaker flours. It is therefore useful for a manufacturer to know something of the strength of the flour he or she is dealing with. Grain variety and the growing conditions of the grain will influence the strength of the resultant flour. However the influence of those and other variables is not completely predictable. Chemical additives can be used to adjust the strength of a flour. For example oxidising agents can be used to decrease the number of thiol groups on gluten proteins and thereby increase gluten cross-linking and kinking.

As well as the influence of flour properties and flour additives, dough properties may be influenced by other ingredients including water, raising agent, mechanically incorporated air, salt, sugar, fat/oil, enzymes and emulsifiers and by temperature. Of all those components the flour properties often exert the greatest influence. The properties of a flour will depend, *inter alia,* on the species of grain from which it is derived (for example wheat flours generally form stronger doughs than do rye flours), the variety of grain (for example durum wheat flours generally form stronger doughs than flours from many other varieties of wheat), and the conditions (weather etc) under which the grain crop was growing.

Flour properties are not entirely predictable, and flours and grains of different perceived properties may vary greatly in price. This makes flour choice and the assessment of flour quality matters of great commercial importance.

It is also desirable to be able to predict the properties of an individual batch of dough. If the properties of a dough batch are not known until the product has been baked and checked then it may be necessary to reject that batch of product. This waste has obvious cost implications.

If the properties of a dough can be determined during mixing, it may be possible to adjust the mixing stage or to supplement the mixture in order to correct a defect or improve final product quality. For example, it may be possible to add additional raising agent if a product with otherwise low raising properties is predicted.

If the properties of a dough can be determined before or during the baking stage, it may be possible to adjust the baking stage in order to correct a defect. For example, it may be appropriate to adjust the oven temperature profile in response to predicted product defect or improve final product quality. Alternatively, an irrecoverable defect may indicate that the product is best discarded before production of the product is complete. Although such a decision will involve unavoidable ingredient wastage, if taken early enough it will limit wastage of labour and of equipment time.

It will be understood that the term "dough" as used herein includes both undeveloped (short) and developed biscuit doughs (for example "Rich Tea"; Trademark, United Biscuits), bread doughs, pasta doughs, snack doughs, and pastries and other shortened doughs (for example cookie doughs). It will also include simple flour and water doughs and flour, water and salt doughs.

In summary, therefore, methods of quantifying objectively the viscoelastic properties of doughs are commercially valuable because they may allow, among other things, for:
1) accurate grading of flour sources,
2) selection of a suitable flour for a specific product,
3) adjustment to a dough recipe in order to compensate for flour deficiencies, for example, by adjusting the quantity of raising agent added, by altering the proofing time or temperature, or by the use of a flour improver or blended flours,
4) adjustment to dough processing or baking, for example by adjusting mixing times or oven temperatures or heating profiles in order to compensate for dough deficiencies.
5) adjustment to the production cycle to develop and assess new products.

Several methods are known in the art for use in the assessment of flour and dough quality.

The Farinograph can be used with a simple flour/water dough to measure the strength of a dough by recording the dough's resistance to a mixing blade during the mixing process. Typically, this resistance increases as the dough develops and then plateaus once development is complete. Over-development, which occurs more easily in weaker doughs, causes a drop in the blade resistance.

The Kieffer Dough and Gluten Extensibility Rig (Developed by Dr Kieffer at the Kurt-Hess Institute, Munich, Germany and available from Stable Micro Systems, Godalming, Surrey, England) analyses the extensibility of a sample of dough by subjecting that sample to uniaxial extension.

Uniaxial extension is a poor model of the dough deformation that occurs during the gas bubble growth which happens during proofing and the earlier stages of baking of a dough product. In order to develop an understanding of dough processes such as bubble formation, bubble growth, bubble bursting, bubble collapse and bubble coalescence, mathematical models of bubble behaviour are used. Such models require as their starting data information concerning the behaviour of a dough when it is subjected to biaxial extension. This is because the dough wall of a bubble is subjected to biaxial forces during phases of bubble behaviour.
Several models of biaxial bubble behaviour are known in the art and it is an aim of the present invention to provide an improved apparatus for providing test data for use in both known mathematical models and those that may be developed in the future in order to take advantage of the increased quality of the test data provided by the apparatus of the invention.

Known mathematical models of biaxial bubble behaviour may be found in for example, van Vliet et al. (1992) "Strain hardening of doughs as a requirement for gas retention", Journal of Texture Studies, 23:439-460; Kokelaar et al. (1996) "Strain hardening properties and extensibility of flour and dough gluten in relation to breadmaking performances", Journal of Cereal Science, 24:199-214; Dobraszcyk and Roberts (1994) "Strain hardening and dough gas cell wall failure in biaxial extension", Journal of Cereal Science, 20:265-274; de Cindio and Correra (1995) "Mathematical modelling of leavened cereal goods", Journal of Food engineering, 24:379-403 and de Cindio et al (2001) "A simple rheological model to predict filled fresh pasta failure during heat treatment", Journal of Food Engineering, 48:7-18.

The Alveograph NG (manufactured by Chopin and available from Tripette and Renaud Export ZI du Val de Seine 20 Avenue Marcellin-Berthelot, F-92390 Villenueve-la Garenne, Ledex, France and described in US 1,468,900) may be used to provide data for models of biaxial bubble behaviour. However the apparatus of the present invention may be used to provide data of a higher quality.

The Dobraszcyk/Roberts (D/R) dough inflation system (available commercially from Stable Micro Systems) was developed from the Chopin Alveograph NG in order to provide test data from biaxial dough extension studies that could be used in predictive models of dough behaviour. Biaxial extension of a dough sample is achieved by air inflation of a sheet of dough. Air is introduced under the dough sheet by displacement of air from a cylinder in the apparatus in response to the movement of a piston in the cylinder. Pressure in the dough macro-bubble (the bubble formed by inflated sheet of dough) is measured by a pressure transducer and the volume of the inflated macro-bubble is calculated as a function of the linear displacement of the piston in the cylinder.

The D/R apparatus and method have a number of drawbacks. These disadvantages serve to limit both the accuracy of the data obtainable and the variety of data which may be gathered. In the D/R apparatus, a sheet of dough is clamped into the apparatus and the sheet is inflated from below by an increasing pressure being applied to the underside of the dough. The pressure above the dough sheet is simply the ambient air pressure. The volume of the macro-bubble is not measured directly but rather it is calculated from the volume of air displaced into it by the cylinder and piston arrangement. This means that the temperature throughout the test must be constant in order to avoid inaccuracies caused by thermal expansion or contraction of the air in the macro-bubble or cylinder. In any case the D/R apparatus has an operating temperature range which is limited to 0 to 40 degrees Celsius because electronic components contained in the apparatus may be damaged if subjected to temperatures outside this range.

Additional inaccuracies may be caused by the fact that the extent of dough deformation, that is the volume of the macro-bubble, is not measured directly but is calculated from a surrogate measurement, that of the volume of air introduced. In order that bubble size and the volume of air introduced can be correlated, it is assumed that any pressure difference between the top and the bottom of the dough sheet has been accommodated by dough sheet deformation (in other words, it is assumed that the bubble is inflated to the fullest possible extent in response to increased internal pressure). That assumption may be reasonable when the macro-bubble inflation rate is low and the dough has sufficient time and flexibility to "take up" any imposed pressure gradient. However, it may be desirable to run an inflation test at a faster rate that is such that the dough does not have sufficient time to respond fully to the imposed pressure. Elongation rate of a dough will strongly depend on that materials properties but rates of up to 0.1s⁻¹ might be typical for certain biscuit doughs. A fast inflation rate may give valuable data on the solid properties of the dough and a low inflation rate may give valuable data on the liquid properties of a dough. Especially valuable data may be that which is obtained from inflation rates during which the dough behaviour is on the borderline between liquid and solid behaviour.

A further drawback of the D/R system is that the dough sample may initially stick to the plate from which it is inflated, especially at increased temperatures. This stickiness requires an initially higher inflation pressure in order to effect dough-sheet un-sticking. This increased initial pressure may give a false measure of dough properties because the pressure will increase without there being a concomitant increase in dough macro-bubble pressure.

Another dough inflation system developed from the Chopin Alveograph NG is that disclosed in US 4,838,081 in which air is introduced under a dough sample quickly initially and then slowly and steadily to stretch the sample disk during which time the air pressure inside the forming dough bubble is measured.

Other technical areas in which knowledge of the viscoelastic properties of materials is important include processing of plastics polymer materials. For example, the viscoelastic properties of raw plastics materials may provide a useful indicator of performance in subsequent processing, for example, injection moulding, blow-moulding, thermoforming or blown film manufacture.

The invention provides a test apparatus for ascertaining properties of a food dough material comprising a chamber in which a chamber pressure is obtained, and holding means for so holding a sheet of the dough material of about 2 cm to about 20 cm in diameter in place relative to said chamber so that a first side of the sheet is exposed to the chamber pressure and a second side of the sheet is exposed to a second pressure, the apparatus further comprising pressure control means for adjusting the chamber pressure to achieve selected pressure parameters, whereby the chamber pressure can be so changed that the sheet can be displaced and further comprising a displacement sensory device for measuring displacement of the sheet. It will be understood that "displacement" includes both inflation of the dough sheet as a result of increasing the pressure obtaining on the first side of the sheet, and displacement of the dough sheet into a chamber in which the pressure has been reduced. Preferably, however, the pressure in the first chamber will be increased.

It will be appreciated that references herein to "controlling" pressure and "control" of pressure mean that the pressure is adjustable to achieve selected pressure parameters, for example, selected absolute pressure values or selected pressure differential across the sheet. Thus, "controlling" of pressure is not intended to include methods in which a gas flow into the chamber is controlled, whilst permitting the pressure to change freely. Pressure control means suitable for effecting pressure control may, for example, comprise a pressure measurement device, a controllable gas introduction device, and a feedback loop comprising a control device for controlling the gas introduction device in dependence on data received from the pressure measurement device.

The apparatus of the invention is for use in the testing of food doughs, for example, bread doughs, pizza doughs, pasta doughs and, especially, biscuit doughs and snack doughs.

Preferably, the test apparatus further comprises a second chamber in which said second pressure is obtained, said holding means being arranged for so holding the sheet of food dough material in place between said first and second chambers so that the first side of the sheet is exposed to the first chamber pressure and the second side of the sheet is exposed to the second chamber pressure, the sheet in use being displacable into at least one of said first and second chambers. It is particularly advantageous for the second side of the sheet also to be enclosed by a chamber (the second chamber), because that permits the conditions to which the sheet is exposed to be more closely monitored and selected. For example, the provision of a chamber on each side of the sheet allows for the gas, and the moisture content thereof, to which the sheet is exposed to be controlled, and for the sheet temperature to be controlled more easily and more precisely, as well as allowing accurate control of the pressure differential across the sheet. Preferably the apparatus comprises means for controlling both the first and second chamber pressures. Preferably the apparatus further comprises a temperature control device for controlling the temperature of the sheet of material. Preferably the first pressure chamber is positioned above the second pressure chamber and the sheet can be positioned substantially horizontally between the first and second chambers. Preferably the sensor device measures the displacement of the sheet from its starting position which is preferably substantially flat. Preferably the displacement sensing device is a laser displacement sensor.

Preferably the walls of the pressure chambers are substantially transparent so that the sheet can be viewed when held in place. For example, the chambers may be constructed of transparent materials or transparent "windows" may be provided.

Preferably the apparatus further comprises means for sensing the temperature and/or pressure in either one or both of the chambers. Absolute pressure may be measured (from which relative pressure can be calculated) alternatively the relative pressure (pressure difference between the chambers) may be measure directly.

Preferably the apparatus further comprises data recording means to record over a period of time data relating to one or more variables selected from deformation, temperature and pressure.

The apparatus may comprise means to manually control inflation of the sheet. Manual control includes control by an operator in response to parameters observed by the operator either directly or in response to instrument data.

Alternatively or additionally, the apparatus comprises means to automatically control inflation of the sheet. Automatic control includes controlling in accordance to a preset or programmable profile or control in utilizing measured parameters.

The apparatus may comprises means to change the pressure in either one or both of the chamber pressures according to a predetermined pressure profile.

The apparatus may comprise means to control the pressure of either or both of the chambers so as to hold the dough sheet substantially flat position in order to prevent or mitigate dripping or sagging of the material.

Alternatively the apparatus may comprise means to control the pressure in either one or both of the chambers so as to give a predetermined rate of sheet inflation.

For certain applications it may be preferable for the apparatus to comprise means to control the pressure in either one or both of the chambers so as to give an inflation rate that allows the material to exhibit properties on or near the boundary between solid and liquid behaviour.

The apparatus may comprise means to inflate the sheet until the inflated sheet bursts or collapses.

For certain applications it may be preferable for the apparatus to provide the relative pressures of the two chambers to be reversed repeatedly so that the sheet oscillates between displacement into the first chamber and inflation into the second chamber. It will be appreciated that such a reversal of relative pressure may be achieved by changing the absolute pressure in either one or both of the chambers.

Preferably the apparatus is arranged so that the extent, rate or type of sheet inflation can be controlled by automatically controlling the gas pressure in one or both of the pressure chambers in response to the position of the sheet as determined by the displacement measuring means.

Preferably the apparatus allows sheet inflation to be carried out at temperatures of more than 40 degrees Celsius. More preferably the apparatus allows sheet inflation to be carried out at temperatures of more than 85 degrees Celsius.

Ideally the apparatus allows sheet inflation to be carried out at a temperature near to those that may be reached during subsequent processing, for example, during baking.

If sheet inflation is to be carried out at an elevated temperature the electronic sensing components are preferably provided in a part of the apparatus that in use of the apparatus is not subjected to temperatures substantially above ambient room temperature, for example, which in use will generally not reach temperatures in excess of 20°C above ambient, and preferably not more than 10°C above ambient. The apparatus is suitable for ascertaining properties of a food dough material, the holding means being suitable for holding a dough sheet.

Preferably the apparatus additionally comprises a microprocessor means that is able to present data on dough properties and make predictions about the baking qualities of that dough as part of a quality control process or a dough or flour grading process.

The apparatus may provide a data output for the manual or automatic control of a step of a baking process.

An example of data outputs is an indication (on a monitor or by means of a lamp) to an operator that the oven temperature should be adjusted. Alternatively such a data output could be arranged to adjust the oven temperature automatically with out operator intervention. Preferably the data output is arranged to be used for the control of an oven temperature profile.

The present invention additionally provides a method of testing a food dough material comprising forming a sheet of the material and subjecting a first side of said sheet to a first pressure and subjecting a second side of said sheet to a second pressure, controlling at least said first pressure, and varying said controlled first pressure so as to achieve selected pressure parameters and give rise to a pressure differential across said sheet wherein said sheet is caused to be displaced and said displacement of said sheet is measured.

Preferably, both the first and second pressures are controlled. Preferably, the temperature of the sheet of material is controlled. Preferably, the sheet is displaced from a substantially flat starting position and the displacement of the sheet from its substantially flat starting position is measured. Preferably, the starting position of the sheet is substantially horizontal. Preferably, sheet displacement is measured by a laser displacement sensor. Preferably, the sheet is visible during displacement through a substantially transparent wall or window of the pressure chamber. Advantageously, at least one parameter selected from pressure and temperature is measured. Preferably, at least one parameter selected from temperature, pressure and displacement is recorded over a period of time.

Inflation of the sheet may be controlled by manual control of at least one of the first and second pressures. Alternatively, inflation of the sheet may be controlled by automatic control of at least one of the first and second pressures.

Inflation of the sheet may be controlled to allow a pre-determined profile of change of either one or both of the first and second pressures.

The pressure of one or both of the chambers may be initially controlled so as to hold the sheet in a substantially flat position in order to prevent or mitigate dripping or sagging of the material.

Pressure in one or both of the chambers may be controlled so as to give an inflation rate that allows the material to exhibit properties on or near the boundary between solid and liquid behaviour.

The inflation of the sheet may be continued until the inflated sheet bursts or collapses. Alternatively, inflation may be stopped at any desired bubble height or volume.

The pressure differential across the sheet may be reversed repeatedly so that the sheet oscillates between displacement into the first chamber and inflation into the second chamber.

At least one parameter selected from the extent, rate and profile of sheet inflation may be controlled by automatically controlling the gas pressure in either one or both of the pressure chambers, and thereby adjusting the pressure differential across the sheet, in response to the position of the sheet as determined by the displacement measuring means.

Preferably, sheet inflation is carried out at temperatures of more than 40 degrees Celsius. More preferably, the apparatus allows sheet inflation to be carried out at temperatures more than 85 degrees Celsius. Ideally inflation is carried out at a temperature near to those that may be reached during a subsequent processing step, for example, in a baking step.

If sheet inflation is effected at elevated temperature those parts of the apparatus that contain electronic sensing components are preferably maintained at a temperature that does not significantly exceed ambient room temperature.

The sheet of material is a sheet of a food dough, for example, a sheet of bread dough, pizza dough, pasta dough, biscuit dough or snack dough. The method of the invention is especially advantageous in the testing of biscuit doughs and snack doughs.

Data collected relating to dough may be used in a quality control process or a dough or flour grading process.

The invention further provides a method of making predictions about the qualities of a dough or flour, comprising testing a dough sample in a method according to the invention and using the data obtained from the dough test in a predictive mathematical model of dough behaviour.

A data output from the dough testing apparatus may be used for the manual or automatic control of a step of a baking process. Preferably the data output is used for the control of an oven temperature profile or any other oven process variable such as pressure at burners or humidity.

The invention further provides a method of testing flour comprising making dough with flour and testing the dough in a method according to the invention.

Whilst, as already mentioned, it is preferred for the apparatus to have first and second chambers, the provision of a second chamber is not essential. Where there is only a first chamber present, the second side of the sheet will be exposed to ambient pressure. Such an arrangement may result in an apparatus which may be of simple construction even though some of the advantages in terms of sophistication of pressure control as present in the preferred two-chamber arrangements are sacrificed.

A significant advantage of the improved apparatus and method of the present invention is that in place of a surrogate measurement dough displacement is measured directly by, for example, macro-bubble height. The invention also provides a system that allows measurements to be taken at a wide range of temperatures including baking temperatures because in one embodiment all temperature sensitive electronic components are outwith the region which is subjected to elevated temperatures.

The present invention also provides a method in which dough testing data obtained in use of a method of the invention is fed backwards or forwards to various stages of a bakery production line. For example, data may be fed backwards to dough mixing equipment or forward to a baking oven.

An embodiment of the apparatus and method of the invention will now be described in detail with reference to the Figures.

Fig. 1 shows a side view of a dough analysis apparatus.

Fig. 2 shows region 9 of the apparatus of Fig. 1 in greater detail.

Figure 3 is a side view of an apparatus of Fig. 1 showing one form of pressure control loop for each of upper and lower chambers.

Fig. 4 diagrammatically shows possible dough analyses.

Fig. 5 shows another apparatus according to the invention, enclosed in a temperature controlled box.

Fig. 6 illustrates by way of a flow chart possible uses of dough analysis data in the control of a bakery product production process.

Fig. 7, 8 and 9 present data obtained from the Example below.

### Test Apparatus

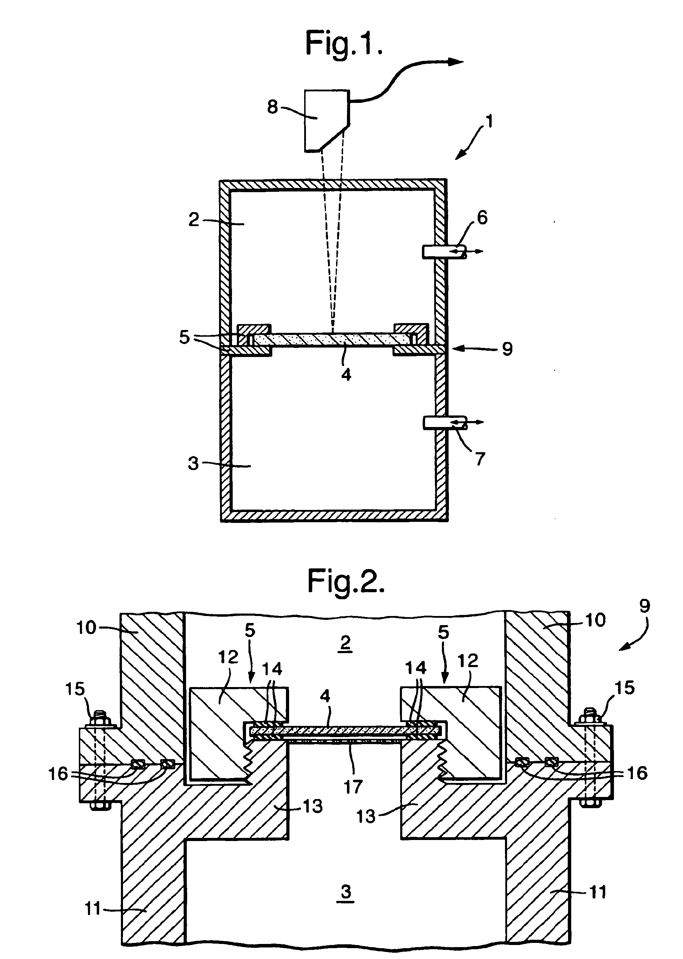

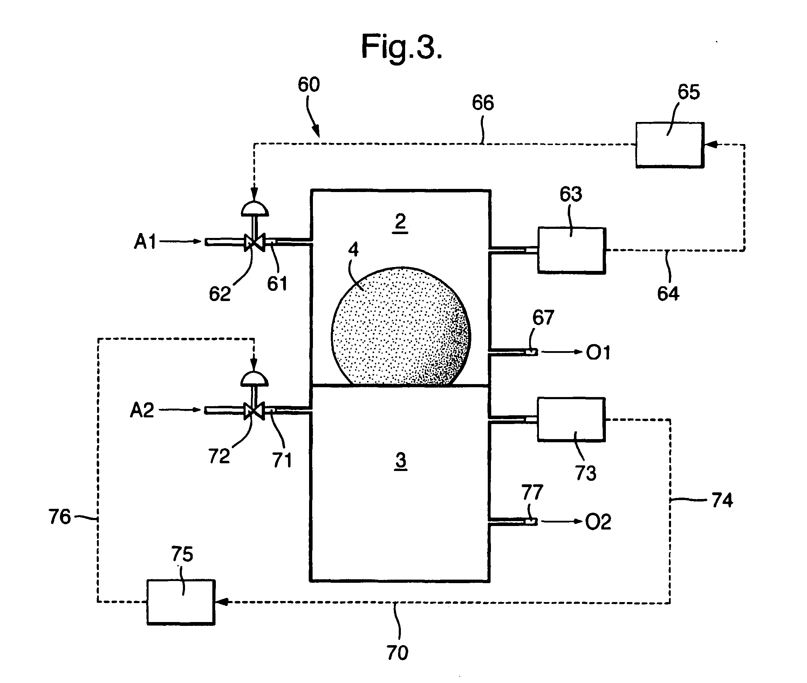

Fig. 1 shows test apparatus 1 comprising two chambers 2 and 3. Between the chambers 2 and 3 (region 9) a dough sample 4 can be fixed in position. The dough sample is held in place by a suitable holding means (shown in simplified form as 5 in Fig. 1). The apparatus is arranged so that when an intact dough sample of a suitable thickness is in place it constitutes a separating wall between chamber 2 and chamber 3. The gas pressure in chambers 2 and 3 is regulated. This is illustrated in Fig. 1 by inlet/outlet pipes 6 and 7 which may be connected to a suitable pressure control means (not shown in Fig. 1). Such a means may comprise a regulated compressed gas cylinder, a gas pump or a piston and cylinder arrangement. The pressure control means must be suitable to generate a different pressure in chamber 2 from that generated in chamber 3. The pressure control means may allow manual adjustment of the pressure or automatic control of the pressure. Ideally the apparatus additionally comprises means to sense the pressure in the two chambers. Such means may be placed inside each chamber or be associated with the pressure control means. Ideally the apparatus comprises a means, for example a mechanical plotter or computer, of recording the pressure as a function of time during the test period. One suitable form of pressure control arrangement is described below with reference to Fig. 3.

The apparatus further comprised means 8 for detecting the position of dough sheet 4. Such means may comprise a video camera, or laser or acoustic position sensing means. Preferably, the position sensing means comprises a laser displacement sensor. Suitable laser displacement sensors are those of the LK range available from Keyence (UK) Limited (Milton Keynes, England). The position sensing means may be connected to a recording means. Preferably the output of the position sensor is connected to a computer that can record the data obtained and optionally use it to control the pressure regulating means. The position sensing means may be positioned in any part of the apparatus from where it can sense the position of the sample. The best view of the sample may be obtained from directly above or directly below the sample. Preferably the position sensing means is positioned directly above the dough sample. The position sensing means may be placed inside or outside either of the pressure chambers 2 and 3. If the position detection means is placed outside the pressure chambers it will be able to avoid any temperature or pressure extremes to which it would otherwise be subjected to and required to withstand. In the embodiment of Fig. 1 laser displacement sensor 8 is fixed above the dough sample and outside of the pressure chambers. The pressure chamber 2 is of a laser-light-permeable material at least in the region of the laser displacement sensor 8 so that the sensor can "see" the sample. Both chambers may be wholly or substantially wholly constructed from clear material such as Perspex (Trademark ICI). Such a construction also has the advantage that the operator can observe the sample directly by eye during the test procedure.

### Material to be tested

The starting dough material may be any type of food dough material. It will be appreciated that the invention is especially suitable for the investigation of doughs that exhibit interesting or complex rheological properties for example viscoelasticity or elastoviscosity.

Typically the sample will be a farinaceous dough. Typically this dough will be made to a recipe of a food product, for example a biscuit recipe. The sample may be a representative portion of material taken from a larger production batch. Alternatively, if the apparatus is to be used to analyse and compare different flours, the dough may be made up to a standard experimental recipe. The dough may be mixed or kneaded by machine or hand as desired. Proofing and relaxation phases may be optionally incorporated into the method.

The sample will be formed into a sheet of material. A sheeting machine or a rolling-pin may be used for this. A test sample may be taken from a production batch before or after sheeting of the product batch. Ideally the material sheet is of substantially even thickness and of approximately circular shape. The most suitable thickness will depend on the type of dough used. A piece of biscuit dough for use in the test apparatus may typically be from about 2 mm to about 6 mm thick, ideally about 3 mm thick. Thicker sheets may be more suitable for bread doughs. Preferably the sheet is circular. The sheet may have a diameter of about 2 cm to about 20 cm, ideally about 10 cm.

Optionally, the sample sheet may be coated with a thin layer of oil, ideally a light mineral oil, in order to prevent it from drying out during the test. This may be especially beneficial if the sample is to be subjected to prolonged elevated temperatures.

### Mounting of sample in apparatus

The sample is mounted in the test apparatus by any means that holds the sample securely in place and which prevents significant leakage of gas from one pressure chamber to the other. Fig. 2 shows region 9 of Fig. 1 in greater detail and illustrates a sample holder which is suitable for use with the invention. The sample holding region 5 fits between the lower regions of the walls 10 of chamber 2 and the upper regions of the walls 11 of chamber 3. In the embodiment shown in Fig. 2 the dough sheet 4 is placed on sample holder 13 and then held in place by clamping ring 12. The sample holder is positioned between chambers 2 and 3. The walls 10 and 11 of chamber 2 and 3 are clamped together using 4 bolts (two shown as 15). Rubber washers 14 and o-rings 16 may be used to ensure a good seal between the various parts of the apparatus.

### Heating of sample

The sample may be heated by heating the surrounding air. However faster heating may be achieved by placing a heating element close to or in contact with the sample. In one preferred embodiment shown in Fig. 2 an electrical heating plate 17 extends between the two chambers and dough sample rests on that. If such an arrangement is used it will be appreciated that holes in the plate will be required in order to allow dough inflation and to reduce dough sticking. Dough temperature may be monitored by any suitable temperature sensing device. A thermocouple may be placed on or in the dough. However this has the draw back of damaging the dough sheet. Such damage can be avoided by using a non contact sensing means such as an infra-red detector or a heat sensitive camera. If the temperature is heated above room temperature the dough may begin to soften and sag. If sagging occurs to such an extent that there is a danger of the dough sheet rupturing under its own weight, it may be necessary to coordinate the heating phase with an increase of pressure below the dough sheet in order that it be supported. This increase in the pressure of chamber 3 may be effected automatically in response to the sensing of sagging or it may be effected manually in response to the sight of sagging. If sagging is to be detected by eye it is advantageous for the whole of a substantial part of the apparatus to be made of a material that is substantially transparent.

### Control of pressure

In Fig. 3 there is shown schematically a test apparatus 1 according to Fig. 1 having two chambers 2, 3, together with suitable pressure control loops 60, 70, which are arranged to effect control of pressure within the chambers 2, 3. An inflating dough sheet is schematically represented by reference numeral 4. Control loop 60, for controlling pressure in chamber 2, includes an air inlet pipe 61 having an electronic control valve 62, for controllably permitting inlet of air flow A1 into chamber 2, and a pressure transducer 63 for measuring pressure within chamber 2. Pressure transducer 63 is connected via communication line 64 to a pressure control unit 65. The pressure control unit 65 is also in communication via communication line 66 with the valve 62. An air outlet pipe 67 is provided for release of air 01 from the chamber 2, and may if desired comprise a control valve (not shown in the drawing) connected to pressure control unit 65.

Control loop 70, for controlling pressure in chamber 3, is of similar general construction to loop 60, comprising air inlet pipe 71 with electronic control valve 72, for permitting inlet of air flow A2 into chamber 3, and a pressure transducer 73 for measuring pressure in chamber 3. Pressure transducer 73 is connected via communication line 74 to a pressure control unit 75, which is in turn in communication with the valve 72 via communication line 76. Air outlet pipe 77 is provided for release of air 02 from chamber 3, and may if desired comprise a control valve (not shown in the drawing) connected to pressure control unit 75.

The pressure control units 65, 75 may comprise manual input means for manual input of a desired pressure for one, or each, of chambers 2, 3. As well, or instead, the control units 65, 75 may comprise automatic control means for control of the pressure, for example, for control of the pressure in accordance with data or algorithms stored in a memory.

In use, the control unit 65 implements a manual or automatic pressure setting by means of monitoring the pressure data received via line 64 from transducer 63 and emitting appropriate control signals via line 66 to valve 62 to effect adjustment thereof for permitting inlet of air until the desired pressure is achieved. On registration of the desired pressure by transducer 63, the control unit 65 emits a "close" instruction to valve 62. Transducer 63 continues to monitor the pressure, and any departure from the desired pressure is then reversed by control unit 65 and valve 62.

Should a decrease in pressure be desired, that may be achieved by opening outlet 67 to permit egress of air, and monitoring the pressure using transducer 63. If fine control of the outflow 01 is not possible in outlet 67, the valve 62 may be used to assist in achieving the decreased target pressure.

The mode of operation of control loop 70 is analogous to that of control loop 60.

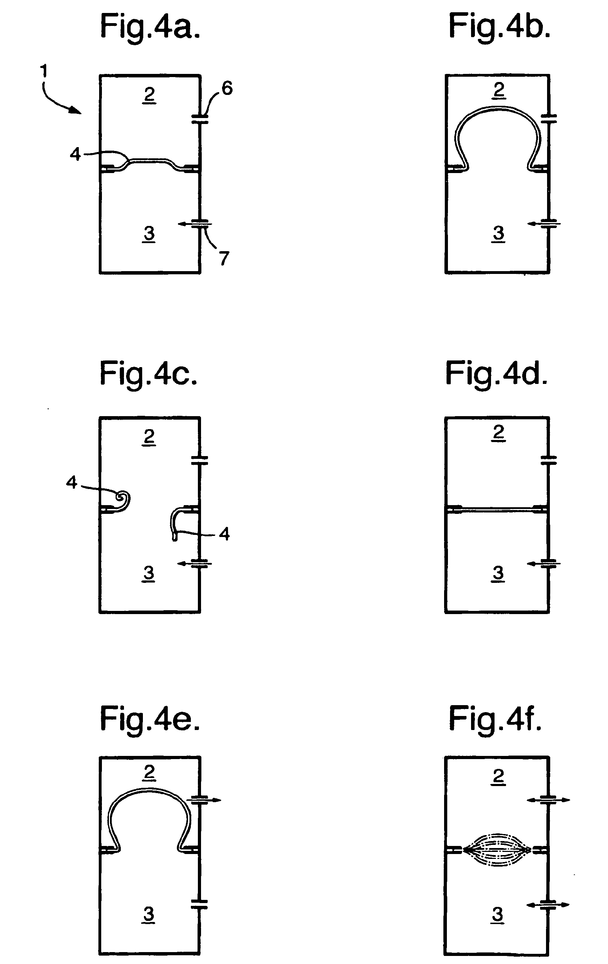

Fig. 4 shows the apparatus of the invention in schematic form with a dough sample 4 in place between pressure chambers 2 and 3. The pressure in each chamber is controllable by pressure control devices represented by reference numerals 6 and 7. The pressure control devices may, for example, be of the kind illustrated in Fig. 3.

Figs. 4a, 4b and 4c illustrate an inflation sequence. The arrow pointing through pressure control device 7 represents air entering chamber 3 and a consequent increase of pressure in that chamber. It will be understood however that an increase of pressure in chamber 3 need only be an increase relative to the pressure in chamber 2. As the pressure in chamber 3 continues to increase, the dough sheet stretches up into a bubble in order to accommodate the pressure change. Fig. 4b shows a more fully inflated bubble than that in Fig. 4a. Eventually, however, the bubble may burst as illustrated in Fig. 4c. Bursting may occur if inflation is too rapid to allow the dough to stretch in order to accommodate the pressure below. Alternatively, even if the relative pressure of chamber 3 increases gradually there will eventually come a point at which the bubble wall will become to thin and the bubble burst.

The increase in relative pressure of the chamber 3 may be controlled manually or automatically. In a manual control system the inflation rate can be judged by eye. In an automatic control system the inflation rate profile may be pre-programmed. For example, the inflation rate may be programmed to give a constant inflation rate or a stepped inflation rate. Alternatively, an automatic control system can produce an inflation rate that causes a pre-determined inflation or deflation rate, the inflation or deflation rate being detected by the displacement sensor means and used to control the pressure control means.

Fig. 4d shows an increased relative pressure in lower chamber 3, but no displacement of the dough sheet 4. It may be the case that under certain circumstances, the dough sheet will have a tendency to sag under its own weight. This may be especially the case when the sheet is heated and the dough softens. Sagging may be prevented by a slight increase in the relative pressure of the lower pressure chamber. This increased pressure may be affected manually in order to correct sagging as judged by the eye or automatically in order to correct sagging as detected by the displacement means. The use of a slight pressure gradient in order to prevent sagging may be especially suitable for use before an inflation test is started. For example, it may be advantageous to keep the dough sheet level during the initial sample-heating phase of a test.

Fig. 4e illustrates that inflation into chamber 2 may be effected by decreasing the pressure in chamber 2 as an alternative, or as well as increasing the pressure in chamber 3 (as shown in Fig. 4b).

Fig. 4f shows a test during which the relative pressures of the two chambers are repeatably exchanged, that is the pressure in chamber 2 goes from being relatively high to relatively low to relatively high to relatively low and so on. The dough sheet 4 is shown to oscillate between a position where it is inflated into chamber 2 and a position where it is inflated in chamber 3. It will be obvious that such an inflation pattern may be readily automated.

### Sensing of dough sheet displacement

The position of dough sheet may be monitored by eye. Such monitoring is especially convenient if the apparatus is wholly or substantially constructed from substantially transparent material. Preferably the position of the dough sheet is monitored automatically. This allows for easy recording of data and easier operation of the apparatus. The dough position may be monitored by a video camera. This may be positioned to "see" the dough sheet substantially edge-on or preferably above the dough sheet. The detection of sheet inflation may be made easier by marking the upper surface, for example with ink. If the dough sheet is marked with two concentric rings, inflation will result in an expansion of the size of the rings and of their distance apart as viewed from above.

A method of detecting sheet movement or inflation that involves marking the dough surface has a number of drawbacks. These include the possibility that the marking substance will affect the dough properties and the requirement for a suitably skilled operator to apply the markings accurately. The need to mark the dough surface is removed if the dough position and inflation is monitored using a displacement sensor device for example an ultrasonic or laser displacement device. A suitable device is one of the LK series of CCD laser displacement sensors available form Keyence Corporation. Such devices are unaffected by surface colour, texture or stray light and can provide resolution of up to 1µm. Laser displacement sensors work by emitting a laser beam which is reflected form the surface to be monitored and is received back by the device. A triangulation measurement system is then used to calculate the distance between the device and the target. Preferably the displacement sensor is placed above the dough sheet in the position shown in Fig. 1. By measuring the distance between the sensor and dough surface, bubble height and therefore bubble volume may be calculated. Laser displacement sensors have the advantage that they may be place at some distance from the dough sheet and do not require any mechanical contact with the sample.

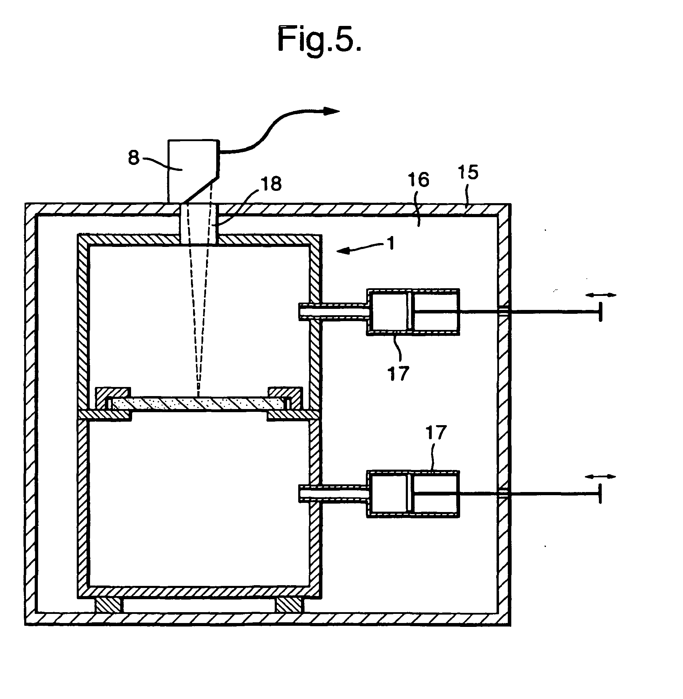

Fig. 5 shows an apparatus wherein the pressure chambers are contained within a temperature controlled box 15. The temperature within this box may be elevated in order to assess dough properties at an elevated temperature. The displacement sensor 8 is placed outside box 15 and so it is not subjected to elevated temperatures that might damage its electronic components. Window 18 allows the displacement sensor to "see" the dough sample 4. In Fig. 1 it is to be assumed that the region of the wall of chamber through which the laser beam (shown as a broken line) must pass is made of laser-transparent material. In Fig. 5 the pressure control devices 6 and 7 are illustrated as being supplied with air from reservoirs 17. These reservoirs are shown as being inside box 15 to allow preheating of reservoir air. Alternatively they could be placed outside box 15. The pressure control devices may be of any suitable kind, for example, of the kind illustrated in Fig. 3 and described above.

### Data obtained from sensor

A laser displacement detector 8 will provide a data output. In its raw form that data will comprise a stream of values of the distance between the sensor and the dough surface. The LK series of laser displacement detectors are capable of giving a reading every 3.5µm seconds, although such a high temporal resolution may not always be required. The data may be displayed on a display module, recorded on a paper plotter or passed to a computer where it may be stored, for example on a data carrier or computer memory, or displayed. A computer will be able to display the data with respect to time or another variable. Ideally the computer is also fed with a signal from temperature and pressure sensors in one or both of the chambers. This will allow temperature, pressure and bubble height/volume to be correlated and plotted against each other or against time. It will also be possible to use sensor data to control the pressure control means. For example data on bubble height may be used to adjust chamber pressure in order to give inflation at a predetermined rate.

### Use of data from analysis of flour and dough

Data on the biaxial extension of a dough may be used in a mathematical model such as those discussed above in order to predict biscuit collapse during baking, product shrinkage and final product density and height. Such modelling will provide valuable information on product quality and will allow for maximisation of quality in future product batches. Additionally because the method of the invention can be used to produce information rapidly, that information may be used to correct defects or otherwise adjust the properties of the dough or product of the batch that has been tested before the completion of baking of that product.

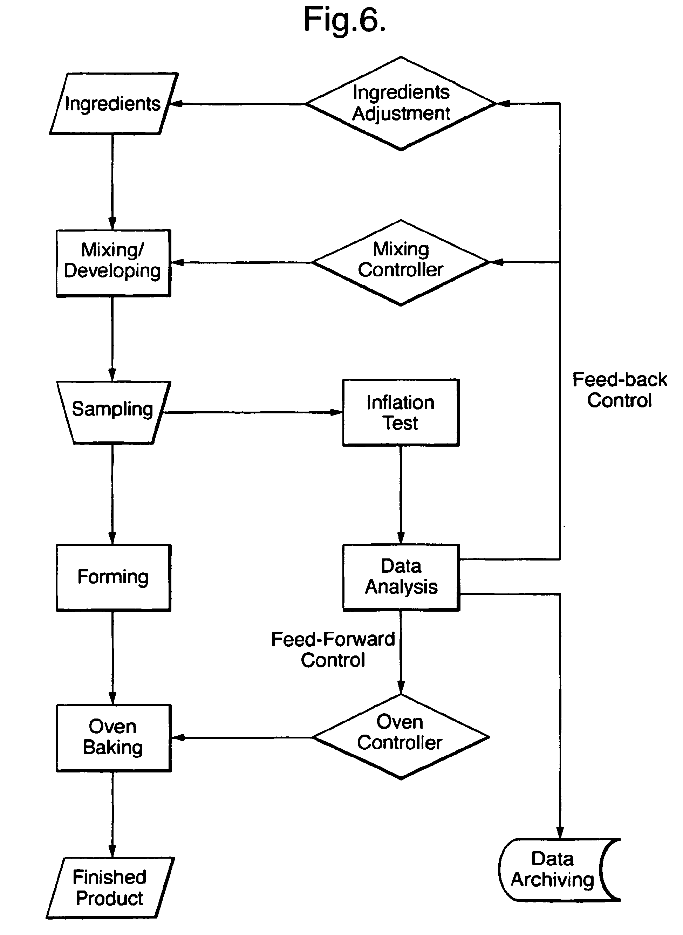

Fig. 6 is a flow chart that shows some possible uses of data obtained from dough analysis. The symbols listed to the left of Fig. 6 show some steps in the process of making a baked product from ingredients to finished product. During mixing a sample is withdrawn and prepared for an inflation test. The test is then performed and the data obtained may be analysed whilst the product has not yet reached a finished product stage. Data analysis may be used to give a prediction of finished product quality. If that quality falls outwith preferred or acceptable values, adjustments may be made to stages of product manufacture upstream of the sampling (feed back control) and/or to stages down-stream of the dough sampling stage (feed forward control). Adjustments may be made to the ingredients mix, extent of mixing, development or proofing time or temperature, product forming stage or oven baking time or temperature profiles. Fig. 6 also shows that all data obtained may be archived. This allows comparisons to be made between product batches and for long term quality trends to be detected.

The following example demonstrates use of the apparatus.

### Example:

This example illustrates the use of the device in the analysis of a developed biscuit dough.
1) Dough was prepared using the following recipe.

| Ingredient | Relative parts |
|---|---|
| Flour | 100 |
| Sugar | 20 |
| Vegetable Oil | 17 |
| Salt | 0.8 |
| Water | 20 |

The starting temperature of the ingredients was 22-24°C.
2) The ingredients were mixed in a z-blade mixer with the mixing bowl surrounded by a 45°C water bath. Ingredients were mixed for 8 minutes.
3) Mixed dough was stored for 20 minutes in a 25°C cabinet. During this storage the dough temperature cooled to 25°C.
4) Samples were sheeted to a 3mm thickness.
5) A 9.8mm circular sample was cut from the dough.
6) The sample was loaded into the sample holder (corresponding to part 5 of Fig. 2) and sealed in place between rubber rings 14 and the upper ring 12 and lower ring 13 of the sample holder.
7) The dough sample was lightly greased on both sides with light mineral oil to prevent dehydration.
8) The sample holder 5 was fixed in place onto the lower half of the apparatus 11.
9) The upper part 10 was put in place and the upper part 10, lower part 11 and sample holder 5 locking in place using 4 bolts. O-ring washers 14 ensured a good seal between all the parts.
10) This test was performed at room temperature. It would however be possible to carry out the test at an alternative temperature. It may, for example, be desirable to carry out the test at oven temperature. If the test is to be carried out at an alternative temperature it is important to allow for sufficient time prior to dough inflation for the material to reach the test temperature. If during this time, melting of the dough increases its tendency to sag under the influence of gravity, a slight positive pressure may be applied to the lower surface of the dough in order to support the sample sheet in a horizontal position and prevent sagging prematurely weakening the dough sheet.
11) Over a period of about 10 seconds to about 2 minutes a bubble of dough was inflated by applying a pressure difference between the upper and lower pressure chambers. This pressure difference was created by introducing air from a compressed air cylinder into pressure chamber 3. The pressure in chamber 3 was regulated to typically 8mm Hg using a Cole-Parmer Vacuum/Pressure Control System EW-68026-52. (Chamber 2 was maintained in pressure equilibrium with the ambient atmosphere.) The pressure difference throughout the test does not normally exceed 15mmHg.
12) Inflation was continued until the bubble ruptured. During inflation the pressure difference between chamber 2 and chamber 3 was measured and recorded by a conventional pressure transducer and the height of the bubble was measured and recorded using a Keyence LK-501 laser displacement sensor in high precision mode and a data logger. Data was recorded once every second, although higher rates are of course possible.

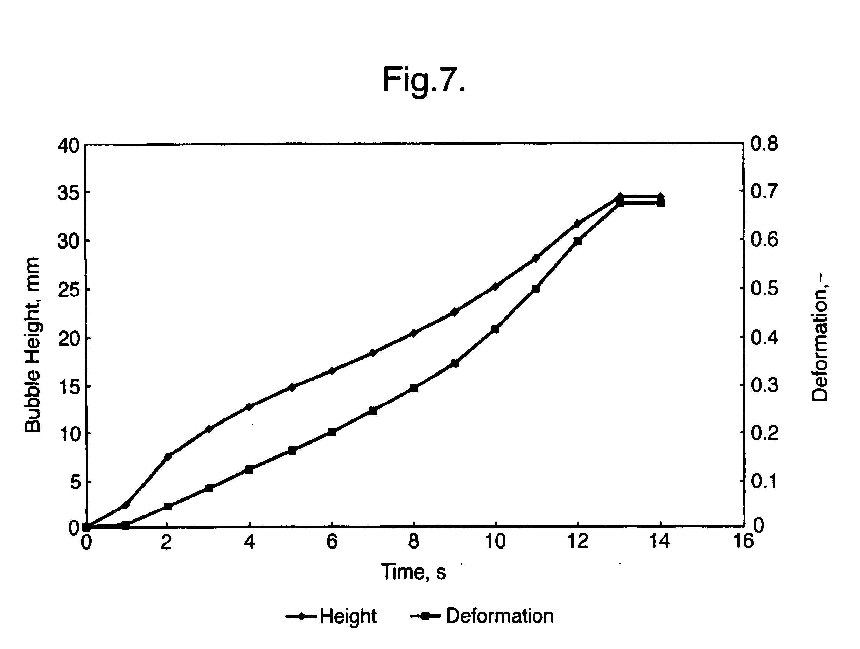

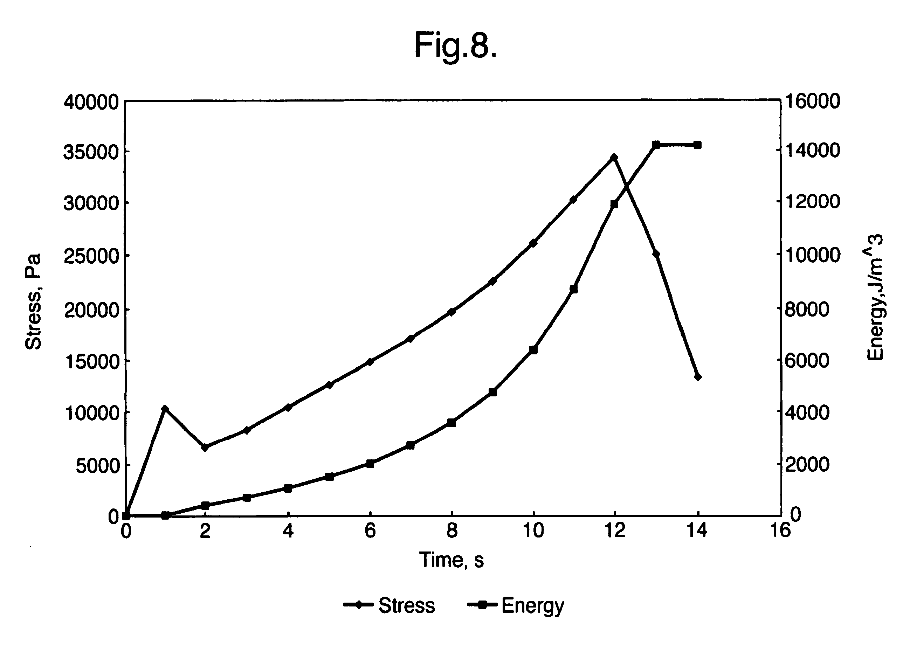

13) Figs. 7, 8 and 9 show data derived from measured values and calculated in accordance with the model given in de Cindio et al (2001) "A simple rheological model to predict filled fresh pasta failure during heat treatment", Journal of Food Engineering, 48:7-18. Fig. 6 shows that deformation increased with time substantially in line with bubble height and Fig. 7 shows calculated stress and energy in the dough during inflation.

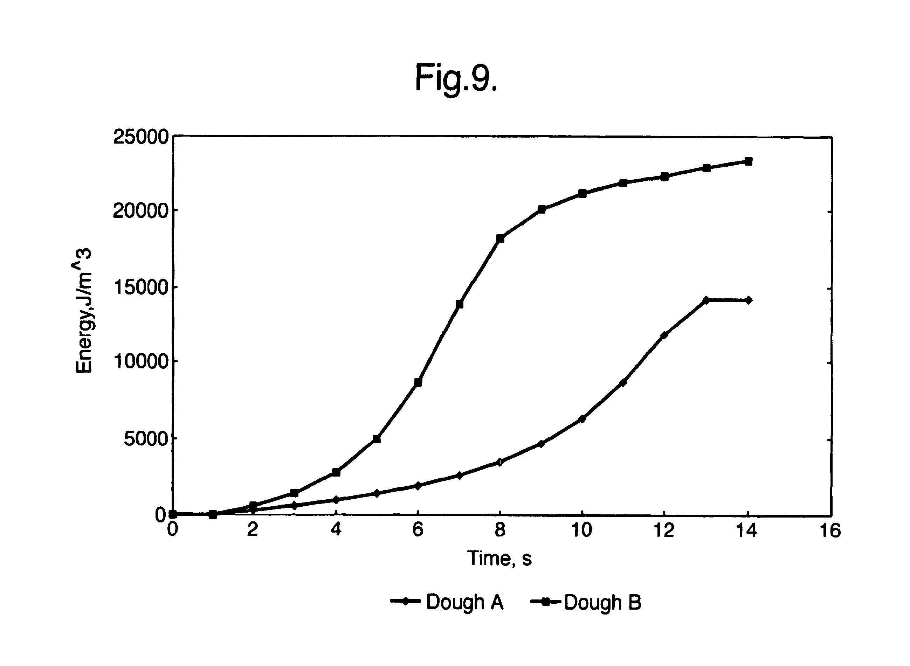

Fig. 9 shows data derived from doughs made from two different flours. The last value in each series is that recorded just before macro bubble rupture. It can be seen that a dough made with flour A exhibits a higher energy value at rupture than does dough made with flour B. This suggests a potentially better performance during baking than flour B. Baking trials confirmed this prediction.
14) Various other rheologic properties may be calculated manually or automatically from the variables recorded parameters. The following formulae are given as examples of properties that may be calculated.
v = ¹/₆ Πh (3a²+ h²)
r = (a²+ h²/2h)
t = t₀ (1+h²/a²) ²
o = pr/2t = p/4 (a²+ h²) ³/ h t₀ a⁴
ε_{H} = In (L/L₀) = ⁻¹/₂ln (t₀/t) = In (1+h²/a²)
ε' = 4h/Π (a²+ h² ) ^{2 d}/_{dt}V
µ_{BE} = σ/ε'
v = Volume of macro bubble
p = Pressure in macro bubble relative to pressure external to macro bubble
h = Macro bubble height
r = Bubble radius
σ = Stress
ε_{H} = Hencky strain
a = Initial sample radius
ε' = Strain rate
t₀ = Initial sample sheet thickness
t = Instantaneous sample sheet thickness
µ_{BE} = Apparent biaxial extensional viscosity

The above Example was also carried out with the increase in pressure difference being effected slowly by manually adjusting pressure valves in order to produce air from a compressed air cylinder into pressure chamber 3 at a rate of 2.5 1 min⁻¹.

## Claims

1. A test apparatus for ascertaining properties of a food dough material comprising a chamber in which a chamber pressure is obtained, and a holding means for so holding a sheet of the dough material of about 2 cm to about 20 cm in diameter in place relative to said chamber so that a first side of the sheet is exposed to the chamber pressure and a second side of the sheet is exposed to a second pressure, the apparatus **characterised by** pressure control means for adjusting the chamber pressure to achieve selected pressure parameters, whereby the chamber pressure can be so changed that the sheet can be displaced, and further comprising a displacement sensory device for measuring displacement of the sheet.

2. An apparatus as claimed in claim 1, in which the displacement sensory device is arranged to measure displacement of the sheet from a starting position in which it is substantially flat.

3. An apparatus as claimed in claim 1 or claim 2, wherein the displacement sensing device is a laser displacement sensor.

4. A test apparatus as claimed in any one of claims 1 to 3, further comprising a second chamber in which said second pressure obtains, said holding means being arranged for so holding the dough sheet in place between said first and second chambers that the first side of the sheet is exposed to the first chamber pressure and the second side of the sheet is exposed to the second chamber pressure, the sheet in use being displaceable into at least one of said first and second chambers.

5. An apparatus as claimed in claim 4, comprising a pressure control device for controlling both the first chamber pressure and the second chamber pressure.

6. An apparatus as claimed in claim 4 or claim 5, further comprising a temperature control device for controlling the temperature of the dough sheet.

7. An apparatus as claimed in any one of claims 4 to 6, wherein the first chamber is positioned above the second chamber in use and the sheet can be positioned , substantially horizontally between the first chamber and second chamber.

8. An apparatus as claimed in any one of claims 4 to 7, further comprising means for sensing the absolute pressure in at least one of the first chamber and second chamber.

9. An apparatus as claimed in any one of claims 4 to 8, further comprising means for sensing the pressure difference between the two chambers.

10. An apparatus as claimed in any one of claims 4 to 9, further comprising means for sensing the temperature of at least one of the first and second chambers.

11. An apparatus as claimed in any one of claims 4 to 10, further comprising data recording means to record over a period of time data relating to one or more variables selected from deformation, temperature and pressure.

12. An apparatus as claimed in any one of claims 4 to 11, comprising means for manual control of inflation of the sheet.

13. An apparatus as claimed in any one of claims 4 to 12, comprising means for automatic control of inflation of the sheet.

14. An apparatus as claimed in any one of claims 4 to 13, comprising means for changing the pressure in one of said first and second chambers according to a predetermined pressure profile.

15. An apparatus as claimed in claim 14, comprising means for changing the pressure in the other of said first and second chambers according to a predetermined pressure profile.

16. An apparatus as claimed in any one of claims 5 to 15, wherein the relative pressures of the first and second chambers can be reversed repeatedly so that the sheet oscillates between inflation into the first chamber and inflation into the second chamber.

17. An apparatus as claimed in any one of claims 4 to 16, in which the arrangement is such that the extent, rate or profile of sheet inflation can be controlled by automatically controlling the gas pressure in one or more of the first and second chambers in response to the position of the sheet as determined by the displacement measuring means.

18. An apparatus as claimed in any one of claims 4 to 17, wherein sheet inflation can be carried out at sheet temperatures of more than 40 degrees Celsius.

19. An apparatus as claimed in any one of claims 4 to 18, wherein electronic sensing components are provided in a part of the apparatus that in use of the apparatus is not subjected to temperatures substantially above ambient room temperature.

20. A test apparatus as claimed in any one of claims 1 to 19, in which inflation of the dough sheet can be carried out at dough temperatures near to those reached during baking.

21. An apparatus as claimed in any one of claims 1 to 20, which comprises a microprocessor means that is able to present data on dough properties, calculate measured values and make predictions about the baking qualities of that dough as part of a quality control process or a dough or flour grading process.

22. A method of testing a food dough material, comprising forming a sheet of the dough material and subjecting a first side of said sheet to a first pressure and subjecting a second side of said sheet to a second pressure, **characterised by** controlling at least said first pressure, and varying said controlled first pressure so as to achieve selected pressure parameters and give rise to a pressure differential across said sheet wherein said sheet is caused to be displaced and said displacement of said sheet is measured.

23. A method as claimed in claim 22, which further comprises recording at least one parameter selected from temperature, pressure or displacement, over a period of time.

24. A method as claimed in any one of claims 22 to 23, wherein at least one or both of the first and second pressures is adjusted so as to give a pre-determined rate of sheet displacement.

25. A method as claimed in any one of claims 22 to 24, wherein the sheet is caused to inflate at a rate that allows the dough material to exhibit properties on or near the boundary between solid and liquid behaviour.

26. A method as claimed in any one of claims 22 to 25, wherein the inflation of the sheet continues until the inflated sheet bursts or collapses.

27. A method as claimed in any one of claims 22 to 25, wherein at least one parameter selected from the extent, rate and profile of sheet displacement is controlled by automatically adjusting the pressure differential across the sheet in response to the position of the sheet as determined by the displacement measuring means.

28. A method as claimed in any one of claims 22 to 27, carried out at sheet temperatures of more than 40 degrees Celsius.

29. A method as claimed in any one of claims 22 to 28, wherein data collected relating to dough is used in a quality control process or a dough or flour grading process.

30. A method of making predictions about the qualities of a dough or flour, comprising testing a dough sample in a method as defined in any of claims 22 to 29, and using the data obtained from the dough test in a predictive mathematical model of dough behaviour.

31. A method as claimed in any of claims 22 to 30, which additionally comprises the step of using a data output from the dough testing apparatus for the manual or automatic control of a step of a baking process, for example, for the control of an oven temperature profile.

32. A method of testing flour comprising making dough with the flour and testing the dough in a method as defined in any one of claims 22 to 31.

## Patentansprüche

1. Prüfvorrichtung zum Ermitteln von Eigenschaften eines Lebensmittel-Teigmaterials, umfassend eine Kammer, in der ein Kammerdruck erzeugt wird, und ein Haltemittel, um so eine Scheibe des Teigmaterials mit einem Durchmesser von etwa 2 cm bis etwa 20 cm in Position zu halten in Bezug auf die Kammer, dass eine erste Seite der Scheibe dem Kammerdruck ausgesetzt ist und eine zweite Seite der Scheibe einem zweiten Druck ausgesetzt ist, wobei die Vorrichtung **gekennzeichnet ist durch** Drucksteuermittel zum Einstellen des Kammerdrucks, um ausgewählte Druckparameter zu erreichen, wodurch der Kammerdruck so veränderbar ist, dass die Scheibe verdrängbar ist, und weiterhin umfassend eine Wegsensorvorrichtung zum Messen der Verdrängung der Scheibe.

2. Vorrichtung nach Anspruch 1, bei der die Wegsensorvorrichtung angeordnet ist, um die Verdrängung der Scheibe von einer Startposition, bei der sie im Wesentlichen flach ist, zu messen.

3. Vorrichtung nach einem der Ansprüche 1 oder 2, wobei die Wegsensorvorrichtung ein Laser-Wegsensor ist.

4. Prüfvorrichtung nach einem der Ansprüche 1 bis 3, weiterhin umfassend eine zweite Kammer, in der der zweite Druck erhalten wird, wobei das Haltemittel angeordnet ist, um so die Teigscheibe in Position zwischen der ersten und zweiten Kammer zu halten, dass die erste Seite der Scheibe dem ersten Kammerdruck ausgesetzt ist und die zweite Seite der Scheibe dem zweiten Kammerdruck ausgesetzt ist, wobei die benutzte Scheibe verdrängbar ist in wenigstens eine der ersten und zweiten Kammern.

5. Vorrichtung nach Anspruch 4, umfassend eine Drucksteuervorrichtung zum Steuern sowohl des ersten Kammerdrucks als auch des zweiten Kammerdrucks.

6. Vorrichtung nach Anspruch 4 oder 5, weiterhin umfassend eine Temperatursteuervorrichtung zum Steuern der Temperatur der Teigscheibe.

7. Vorrichtung nach einem der Ansprüche 4 bis 6, wobei die erste Kammer oberhalb der benutzten zweiten Kammer positioniert ist und die Scheibe im Wesentlichen horizontal zwischen der ersten und der zweiten Kammer positionierbar ist.

8. Vorrichtung nach einem der Ansprüche 4 bis 7, weiterhin umfassend Mittel zum Anzeigen des absoluten Drucks in mindestens einer der ersten und zweiten Kammer.

9. Vorrichtung nach einem der Ansprüche 4 bis 8, weiterhin umfassend Mittel zum Anzeigen der Druckdifferenz zwischen den beiden Kammern.

10. Vorrichtung nach einem der Ansprüche 4 bis 9, weiterhin umfassend Mittel zum Anzeigen der Temperatur von mindestens einer der ersten und zweiten Kammern.

11. Vorrichtung nach einem der Ansprüche 4 bis 10, weiterhin umfassend Datenaufzeichnungsmittel zum Aufzeichnen von Daten betreffend eine oder mehrere Variablen, ausgewählt aus Deformation, Temperatur und Druck, über eine Zeitdauer.

12. Vorrichtung nach einem der Ansprüche 4 bis 11, umfassend Mittel zum manuellen Steuern eines Aufblähens der Scheibe.

13. Vorrichtung nach einem der Ansprüche 4 bis 12, umfassend Mittel zum automatischen Steuern des Aufblähens der Scheibe.

14. Vorrichtung nach einem der Ansprüche 4 bis 13, umfassend Mittel zum Ändern des Drucks in einer der ersten und zweiten Kammern gemäß einem vorbestimmten Druckprofil.

15. Vorrichtung nach Anspruch 14, umfassend Mittel zum Ändern des Drucks in der anderen der ersten und zweiten Kammern gemäß einem vorbestimmten Druckprofil.

16. Vorrichtung nach einem der Ansprüche 5 bis 15, wobei die relativen Drücke der ersten und zweiten Kammern wiederholt umkehrbar sind, so dass die Scheibe zwischen einer Aufblähung in die erste Kammer und einer Aufblähung in die zweite Kammer oszilliert.

17. Vorrichtung nach einem der Ansprüche 4 bis 16, bei der die Anordnung derart ist, dass der Umfang, der Grad oder das Profil der Scheibenaufblähung steuerbar ist durch automatisches Steuern des Gasdrucks in einer oder mehreren der ersten und zweiten Kammern als Antwort auf die Position der Scheibe, wie von den Mitteln zum Messen der Verdrängung bestimmt.

18. Vorrichtung nach einem der Ansprüche 4 bis 17, wobei die Scheibenaufblähung bei Scheibentemperaturen von mehr als 40 °C durchführbar ist.

19. Vorrichtung nach einem der Ansprüche 4 bis 18, wobei elektronische Anzeigekomponenten vorgesehen sind in einem Teil der Vorrichtung, der bei Verwendung der Vorrichtung nicht Temperaturen im Wesentlichen über der äußeren Raumtemperatur ausgesetzt ist.

20. Prüfvorrichtung nach einem der Ansprüche 1 bis 19, bei der die Aufblähung der Teigscheibe bei Teigtemperaturen nahe den beim Backen erreichten durchführbar ist.

21. Vorrichtung nach einem der Ansprüche 1 bis 20, die ein Mikroprozessormittel umfasst, das fähig ist, Daten der Teigeigenschaften anzuzeigen, gemessene Werte zu berechnen und Voraussagen über die Backqualitäten des Teigs zu treffen als ein Teil eines Qualitätssteuerungsprozesses oder eines Teig- oder Mehlaufbereitungsprozesses.

22. Verfahren zum Prüfen eines Lebensmittel-Teigmaterials, umfassend das Bilden einer Scheibe des Teigmaterials und das Aussetzen einer ersten Seite der Scheibe einem ersten Druck und das Aussetzen einer zweiten Seite der Scheibe einem zweiten Druck, **gekennzeichnet durch** das Steuern mindestens des ersten Drucks und Ändern des gesteuerten ersten Drucks derart, um ausgewählte Druckparameter zu erreichen und ein Druckdifferential über diese Scheibe zu bewirken, wobei eine Verdrängung der Scheibe bewirkt wird und die Verdrängung der Scheibe gemessen wird.

23. Verfahren nach Anspruch 22, das weiterhin das Aufzeichnen mindestens eines Parameters, ausgewählt aus Temperatur, Druck oder Verdrängung, über eine Zeitdauer umfasst.

24. Verfahren nach einem der Ansprüche 22 bis 23, wobei mindestens einer oder beide der ersten und zweiten Drücke eingestellt wird derart, um ein vorbestimmtes Maß an Scheibenverdrängung zu erzielen.

25. Verfahren nach einem der Ansprüche 22 bis 24, wobei ein Aufblähen der Scheibe erzielt wird in einem Grad, das es dem Teigmaterial ermöglicht, Eigenschaften an oder nahe der Grenze zwischen festem und flüssigem Verhalten aufzuweisen.

26. Verfahren nach einem der Ansprüche 22 bis 25, wobei die Aufblähung der Scheibe sich fortführt, bis die aufgeblähte Scheibe reißt oder zusammenbricht.

27. Verfahren nach einem der Ansprüche 22 bis 25, wobei mindestens ein Parameter, ausgewählt aus Umfang, Grad und Profil der Scheibenverdrängung, gesteuert wird durch automatisches Einstellen des Druckdifferentials über die Scheibe in Antwort auf die Position der Scheibe, wie von den Mitteln zum Messen der Verdrängung bestimmt.

28. Verfahren nach einem der Ansprüche 22 bis 27, ausgeführt bei Scheibentemperaturen von mehr als 40 °C.

29. Verfahren nach einem der Ansprüche 22 bis 28, wobei gesammelte den Teig betreffende Daten in einem Qualitätssteuerungsprozess oder in einem Teig- oder Mehlaufbereitungsprozess verwendet werden.

30. Verfahren zum Treffen von Vorhersagen über die Qualitäten eines Teigs oder Mehls, umfassend das Prüfen einer Teigprobe in einem Verfahren, wie in einem der Ansprüche 22 bis 29 bestimmt, und Verwenden der von der Teigprüfung erhaltenen Daten in einem vorhersagenden mathematischen Modell des Teigverhaltens.

31. Verfahren nach einem der Ansprüche 22 bis 30, das zusätzlich den Schritt des Verwendens eines Datenausgangs von der Teig-Prüfvorrichtung für das manuelle oder automatische Steuern eines Schrittes des Backprozesses, beispielsweise zum Steuern eines Ofentemperaturprofils, umfasst.

32. Verfahren zum Prüfen von Mehl, umfassend das Herstellen von Teig mit dem Mehl und das Prüfen des Teigs in einem Verfahren, wie in einem der Ansprüche 22 bis 31 bestimmt.

## Revendications

1. Dispositif de test destiné à vérifier des propriétés d'un matériau de pâte alimentaire comprenant une chambre dans laquelle une pression de chambre est obtenue et un moyen de maintien destiné à maintenir une feuille du matériau de pâte d'environ 2 cm à environ 20 cm de diamètre en place par rapport à ladite chambre de sorte qu'un premier côté de la feuille est exposé à la pression de chambre et qu'un deuxième côté de la feuille est exposé à une deuxième pression, le dispositif étant **caractérisé par** un moyen de commande de pression destiné à ajuster la pression de chambre pour obtenir des paramètres de pression sélectionnés, grâce à quoi la pression de chambre peut être changée de sorte que la feuille puisse être déplacée, et comprenant en outre un dispositif de détection de déplacement destiné à mesurer le déplacement de la feuille.

2. Dispositif selon la revendication 1, dans lequel le dispositif de détection de déplacement est agencé pour mesurer un déplacement de la feuille à partir d'une position de début dans laquelle elle est sensiblement plate.

3. Dispositif selon la revendication 1 ou la revendication 2, dans lequel le dispositif de détection de déplacement est un capteur de déplacement à laser.

4. Dispositif de test selon l'une quelconque des revendications 1 à 3, comprenant en outre une deuxième chambre dans laquelle ladite deuxième pression permet, ledit moyen de maintien étant agencé pour maintenir la feuille de pâte en place entre lesdites première et deuxième chambres de telle façon que le premier côté de la feuille soit exposé à la première pression de chambre et que le deuxième côté de la feuille soit exposé à la deuxième pression de chambre, que la feuille utilisée puisse être déplacée dans au moins l'une chambre desdites première et deuxième chambres.

5. Dispositif selon la revendication 4, comprenant un dispositif de commande de pression destiné à commander à la fois la première pression de chambre et la deuxième pression de chambre.

6. Dispositif selon la revendication 4 ou la revendication 5, comprenant en outre un dispositif de commande de température destiné à commander la température de la feuille de pâte.

7. Dispositif selon l'une quelconque des revendications 4 à 6, dans lequel la première chambre est positionnée au-dessus de la deuxième chambre en utilisation et la feuille peut être positionnée globalement horizontalement entre la première chambre et la deuxième chambre.

8. Dispositif selon l'une quelconque des revendications 4 à 7, comprenant en outre un moyen destiné à détecter la pression absolue dans au moins l'une de la première chambre et de la deuxième chambre.

9. Dispositif selon l'une quelconque des revendications 4 à 8, comprenant en outre un moyen destiné à détecter la différence de pression entre les deux chambres.

10. Dispositif selon l'une quelconque des revendications 4 à 9, comprenant en outre un moyen destiné à détecter la température d'au moins l'une des première et deuxième chambres.

11. Dispositif selon l'une quelconque des revendications 4 à 10, comprenant en outre un moyen d'enregistrement de données destiné à enregistrer sur un intervalle de temps des données associées à une ou plusieurs variables sélectionnées à partir d'une déformation, de la température et de la pression.

12. Dispositif selon l'une quelconque des revendications 4 à 11, comprenant un moyen destiné à une commande manuelle de gonflement de la feuille.

13. Dispositif selon l'une quelconque des revendications 4 à 12, comprenant un moyen destiné à une commande automatique de gonflement de la feuille.

14. Dispositif selon l'une quelconque des revendications 4 à 13, comprenant un moyen destiné à changer la pression dans l'une desdites première et deuxième chambres conformément à un profil de pression prédéterminé.

15. Dispositif selon la revendication 14, comprenant un moyen destiné à changer la pression dans l'autre desdites première et deuxième chambres conformément à un profil de pression prédéterminé.

16. Dispositif selon l'une quelconque des revendications 5 à 15, dans lequel les pressions relatives des première et deuxième chambres peuvent être inversées de façon répétée de sorte que la feuille oscille entre un gonflement dans la première chambre et un gonflement dans la deuxième chambre.

17. Dispositif selon l'une quelconque des revendications 4 à 16, dans lequel l'agencement est tel que l'étendue, la vitesse ou le profil du gonflement de la feuille peuvent être commandés en commandant automatiquement la pression de gaz dans une ou plusieurs des première et deuxième chambres en réponse à la position de la feuille comme déterminé par le moyen de mesure de déplacement.

18. Dispositif selon l'une quelconque des revendications 4 à 17, dans lequel le gonflement de la feuille peut être mis en oeuvre à des températures de feuille de plus de 40 degrés Celsius.

19. Dispositif selon l'une quelconque des revendications 4 à 18, dans lequel des composants de détection électroniques sont prévus dans une partie du dispositif qui, lors de l'utilisation du dispositif, n'est pas soumise à des températures considérablement au-dessus de la température ambiante.

20. Dispositif de test selon l'une quelconque des revendications 1 à 19, dans lequel le gonflement de la feuille de pâte peut être mis en oeuvre à des températures de pâte proches de celles atteintes au cours de la cuisson.

21. Dispositif selon l'une quelconque des revendications 1 à 20, lequel comprend un moyen de microcalculateur qui est capable de présenter des données concernant des propriétés de pâte, de calculer des valeurs mesurées et de réaliser des prédictions en ce qui concerne les qualités de cuisson de cette pâte en tant que partie d'un processus de commande de qualité ou d'un processus de classement de pâte ou de farine.

22. Procédé de test d'un matériau de pâte alimentaire, comprenant la mise en forme d'une feuille du matériau de pâte et la soumission d'un premier côté de ladite feuille à une première pression et la soumission d'un deuxième côté de ladite feuille à une deuxième pression, **caractérisé par** la commande d'au moins ladite première pression et la variation de ladite première pression commandée de manière à obtenir des paramètres de pression sélectionnés et faire apparaître une pression différentielle à travers ladite feuille où ladite feuille est amenée à être déplacée et ledit déplacement de ladite feuille est mesuré.

23. Procédé selon la revendication 22, lequel comprend en outre l'enregistrement d'au moins un paramètre sélectionné à partir de la température, de la pression ou du déplacement, sur un intervalle de temps.

24. Procédé selon l'une quelconque des revendications 22 à 23, dans lequel au moins l'une des première et deuxième pressions, ou bien les deux, est ajustée de manière à donner une vitesse prédéterminée du déplacement de la feuille.

25. Procédé selon l'une quelconque des revendications 22 à 24, dans lequel la feuille est amenée à gonfler à une vitesse qui permet que le matériau de pâte présente des propriétés sur ou proches de la limite entre un comportement de solide et de liquide.

26. Procédé selon l'une quelconque des revendications 22 à 25, dans lequel le gonflement de la feuille se poursuit jusqu'à ce que la feuille gonflée éclate ou s'écrase.

27. Procédé selon l'une quelconque des revendications 22 à 25, dans lequel au moins un paramètre sélectionné à partir de l'étendue, de la vitesse et du profil du déplacement de la feuille est commandé en ajustant automatiquement la pression différentielle à travers la feuille en réponse à la position de la feuille, comme déterminé par le moyen de mesure de déplacement.

28. Procédé selon l'une quelconque des revendications 22 à 27, exécuté à des températures de feuille de plus de 40 degrés Celsius.

29. Procédé selon l'une quelconque des revendications 22 à 28, dans lequel les données recueillies se rapportant à la pâte sont utilisées dans un processus de commande de qualité ou un processus de classement de pâte ou de farine.

30. Procédé de réalisation de prédictions en ce qui concerne les qualités d'une pâte ou d'une farine, comprenant le test d'un échantillon de pâte dans un procédé tel que défini dans l'une quelconque des revendications 22 à 29 et l'utilisation des données obtenues à partir du test de pâte dans un modèle mathématique prédictif du comportement de la pâte.

31. Procédé selon l'une quelconque des revendications 22 à 30, lequel comprend de façon supplémentaire l'étape consistant à utiliser des données fournies en sortie du dispositif de test de pâte pour la commande manuelle ou automatique d'une étape d'un processus de cuisson, par exemple, pour la commande d'un profil de température de four.

32. Procédé de test de farine comprenant la réalisation d'une pâte avec la farine et le test de la pâte dans un procédé tel que défini dans l'une quelconque des revendications 22 à 31.
